# EUROPEAN PATENT SPECIFICATION

(11) **EP 1 564 230 B1**
(45) Date of publication and mention of the grant of the patent: **09.03.2011**
(21) Application number: 05009167.7
(22) Date of filing: 25.08.2001
(51) Int. Cl.: C08F 220/56, A61L 27/16, A61L 27/52, C08L 33/26

(54) **Polyacrylamide hydrogel and its use as an endoprosthesis**
Polyacrylamid-Hydrogel und seine Verwendung als Endoprothese
Hydrogel de polyacrylamide et son utilisation comme endoprothèse

(30) Priority: 25.08.2000 DK 200001262
(43) Date of publication of application: 17.08.2005
(62) Divisional of application: 01960207.7
(73) Proprietor: Contura A/S, 2860 Søborg (DK)
(72) Inventor: Lessel, Robert, 2605 Brondby (DK); Schmidt, Richard, 2950 Vedbaek (DK)
(74) Representative: Plougmann & Vingtoft A/S

(56) References cited:
- EP-A- 0 727 232
- EP-A- 0 742 022
- WO-A-01/49336
- WO-A-99/10021
- US-A- 4 344 193

## Description

### FIELD OF INVENTION

The present invention relates to a novel polyacrylamide hydrogel cross-linked polyacrylamide. The hydrogel is obtainable by combining the acrylamide and methylene bis-acrylamide in a specific ratio so as to confer physical properties to the hydrogel. The present invention further relates to an endoprosthesis for the treatment of arthritis.

### BACKGROUND OF THE INVENTION

Natural and synthetic polymers such as collagen, soya, glycerol, silicone, polyvinylpyrolidone and hyaluronic acid have been utilised as endoprostheses. Materials used for endoprostheses generally try to imitate the natural soft tissue and are intended to be safe to the health of the patient.

Polyacrylamide gels have also been disclosed. US 5,798,096 relates to a biocompatible hydrogel containing 3.5 to 6.0% cross-linked polyacrylamide. However, US 5,798,096 teaches that concentrations below 3.5% make the hydrogel unstable.

GB 2114578 relates to a polyacrylamide gel for medical and biological purposes containing 3 to 28% polyacrylamide with the remainder of the mass of the gel comprised of a physiological solution.

US 5,658,329 relates to an implantable endoprosthesis comprising a shell filled with a polyacrylamide gel comprising 2 to 20% polyacrylamide by weight and a viscosity range of 15 to 75 Pas.

Formacryl^{®} polyacrylamide is a soft-tissue endoprosthesis consisting of 5% reticulated polyacrylamide polymer and 95% apyrogenic water commercialised as an injectable device for medical and dental use to correct congenital or acquired deficits such as wrinkles, lines and scars. It is to be implanted with a syringue in the hypodermis.

US 5,306,404 relates to a process for preparing polyacrylamide gel plates for electrophoresis.

WO 99/10021 relates to an injectable, biocompatible hydrogel comprising 0.5 to 10% polyacrylamide and an antibiotic or antiseptic. WO 99/10021 is directed to the solving the problem of sappuration and rejection of the gel in its use an endoprosthesis.

EP 0 742 022 discloses a biocompatible hydrogel containing 3.5-9.0 wt% acrylamide crosslinked with methylene bis-acrylamide and saline solution for correcting cosmetic and functional defects of human organisms by injection.

The application WO 01/49336 discloses a biocompatible hydrogel for endoprostheses comprising 2-15 wt% polyacrylamide cross-linked with methylene bis-acrylamide.

### SUMMARY OF THE INVENTION

The generally invention relates to the biostable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution The bio-stable hydrogel typically has a molecular weight between 0.01 x 10⁶ and 20 x 10⁶ . The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. The hydrogel is useful as an endoprosthetic amount, said gel tailored to the defect it seeks to correct.

One object of the invention is to provide a polyacrylamide hydrogel for use as a prosthetic device for supplementing, augmenting or replacing cartilage in the intra-articular cavity of a joint. The soft material has at least two advantageous features in that the material is firstly both biocompatible and biostable; and secondly, the material is mechanically resilient and does not bead, tear, shred or disintegrate readily upon mechanical stress. The material may be injected or implanted and manipulated so as to distribute the support provided by the material uniformly or according to the needs of the patient. The hydrogel also provides lubrication to the joint and the pre-existing cartilage.

A central object of the invention is to provide a hydrogel for use in the in the treatment or prevention of arthritis said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

A further aspect of the invention relates to the use of a hydrogel comprising about 0.5 to 25% by weight polyacrylamide, based on the total weight of the hydrogel, for the preparation of an endoprosthesis for alleviation or prevention of symptoms associated with arthritis.

Furthermore, providing a method of treating or preventing arthritis comprising administering a hydrogel to a mammal said hydrogel comprising 0.5 to 25% by weight polyacrylamide, based on the total weight of the hydrogel is further object of the invention.

Another aspect of the invention relates to a prosthetic device for the treatment of arthritis, wherein the device comprises a polyacrylamide hydrogel comprising 0.5 to 25% by weight polyacrylamide, based on the total weight of the hydrogel, said device administered to the intra-articular cavity of joint. Alternatively defined, the prosthetic device of the invention is for augmenting or replacing cartilage in the intra-articular cavity of a joint, said device comprises a polyacrylamide hydrogel comprising 0.5 to 25% by weight polyacrylamide, based on the total weight of the hydrogel.

### DETAILED DESCRIPTION OF THE INVENTION

### Hydrogels and Their Preparation

The success of plastic or reconstructive surgery depends to a great extent on the physical properties of the materials utilised. They must most certainly be biocompatible, stable and non-toxic but they must also have physical properties that mimic the bodily tissue they are replacing, as in reconstructive surgery, or mimic the bodily tissue in the proximity of the endoprosthesis, as in cosmetic surgery.

Materials such as collagen are resorbed into the body over short periods of time. Silicone and Soya have encountered serious safety issues. There is currently a need for a safe, stable, biocompatible material that possesses the physical properties to mimic soft tissue. The present inventors have surprisingly found that a polyacrylamide hydrogel comprising less than 3.5% polyacrylamide, based on the total weight of the hydrogel, is an effective endoprosthesis with advantageous physical properties. Contrary to US 5,798,096, the polyacrylamide gel is stable. The hydrogel is cross-linked with methylene bis-acrylamide to a degree such that the endoprosthesis prepared from said hydrogel possesses advantageous physical characteristics. The hydrogel according to the present invention is a new chemical entity as indicated by its novel and advantageous physical characteristics. These secondary characteristics are indicative that the degree of cross-linking in the hydrogel of the present invention differs greatly from polyacrylamide hydrogels prepared by disclosed processes. This degree of cross-linking is a critical contributor to its physical properties.

The present investigators have provided a bio-stable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. The bio-stable hydrogel typically has a molecular weight between 0.01 x 10⁶ and 20 x 10⁶. The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

The hydrogel comprises less than 3.5% polyacrylamide preferably comprising at least 0.5%, such as at least 1 %, preferably at least 1.5% polyacrylamide, such as at least 1.6% polyacrylamide by weight, based on the total weight of the hydrogel.

The hydrogel comprising less than 3.5% polyacrylamide are chemically stable and bio-stable but may be very fluid such that they may be characterised in that it has a complex viscosity not less than 2 Pas, such as not less than 3, 4 or 5 Pas. In a suitable embodiment, the hydrogel comprising less than 3.5% polyacrylamide is characterised in that it has complex viscosity from about 2 to 90, such as 5 to 80 Pas, preferably from about 6 to 76, such as from about 6 to 60, 6 to 40, 6 to 20, such as 6 to 15 Pas.

The hydrogel comprising less than 3.5% polyacrylamide has elastic properties in that the hydrogel may be characterised in that it has elasticity module of not less than 10 Pa, such as not less than 20, 25, 30, 31, 32, 33, 34 or 35 Pa, such as not less than 38 Pa. Typically, the hydrogel has an elasticity module from about 10 to 700 Pa, such as about 35 to 480 Pa.

These rheological features are in due in part to the degree of cross-linking and to the degree of swelling of the hydrogel. The hydrogel comprising less than 3.5% polyacrylamide may be characterised in that the cross-linked polyacrylamide is to such as degree so as to have an efficient cross-linking density of about 0.2 to 0.5%, preferably about 0.25 to 0.4%.

The cross-linking density is in turn due in part to the molar ratio between the acrylamide and methylene-bis-acrylamide. Typically said ratio is in the range 175:1 to 800:1, such as from 225:1 to 600:1, preferably from 250:1 to 550:1, most preferably from 250:1 to 500:1. The absolute and relative amount of the redox agent (TEMED) and the initiator also influence the degree of cross-linking. As can be seen from Tables 1, 2, 3, 4, the present investigators have adjusted these parameters to influence the rheological properties of the hydrogel.

The biocompatible hydrogels of the invention comprising less than 3.5% polyacrylamide may be suitably characterised, at least in part, by one or more of the following features: i) a cross-linking density of 0.2% to 0.5 %; ii) an elasticity modulus (G') of 10 to 700 Pa; iii) a complex viscosity of 2 to 90 Pa s; iv) a dry matter content of less than 3.5% such as less than 3.4, such as less than 3.3, such as less than 3.2, such as less than 3.1, such as less than 3.0, such as less than 2.9, such as less than 2.8, such as less than 2.7, such as less than 2.6 Pa s; v) a refractive index between 1.33 and 1.34.

As stated, in all aspects of the invention wherein the hydrogel comprises less than 3.5% polyacrylamide by weight, based on the total weight of the hydrogel, the hydrogel further comprising at least 95% pyrogen-free water or saline solution. In all embodiments wherein the hydrogel further comprises saline solution, the hydrogel preferably comprises less than 3 % polyacrylamide by weight, based on the total weight of the hydrogel.

The hydrogel of the invention is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

The hydrogel comprises pyrogen-free water or saline solution. The combining of the reagents and the casting of the gel may therefore be done in pyrogen-free water or saline solution. The use of saline solution will clearly increase the total solid weight content of the hydrogel but does not significantly influence the polyacrylamide content during the polymerisation reaction.

A suitable saline solution has an osmolarity similar to that of interstitial fluid. Suitable saline solutions include but are not limited to the group selected from 0.25- 1% aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is an about 0.8- 1 % aqueous sodium chloride solution, such as a 0.8, 0.9 or 1% aqueous sodium chloride solution.

As stated, pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm. In the method of the invention, the washing step comprises swelling the product for 50 to 250 hours, more typically for 70 to 200 hours.

The hydrogel comprising less than 3.5% polyacrylamide was surprisingly found to be stable at very low solid weight content, contrary to the teachings of US 5,798,096.

Polyacrylamide hydrogels with a solid-weight content of 0.5% have been prepared by the present investigators. Preferred embodiments of the hydrogel of the invention comprise at least 0.5%, such as at least 1 %, preferably at least 1.5% polyacrylamide, such as at least 1.6% polyacrylamide by weight, based on the total weight of the hydrogel.

The hydrogel is suitably a composite of cross-linked polyacrylamide chains and pyrogen-free water. Water contained in the hydrogel as part of a composite is loosely bound with the polymer chains. When present in a body, some of the water molecules move into the tissue by osmosis resulting in a smoothing out of the affected skin surface. In the embodiment wherein the hydrogel comprises saline solution, the iso-osmolarity of the saline solution with interstitial fluid minimises immune responses.

As mentioned, the physical properties of the hydrogel are influenced in part by the degree of cross-linking. The degree of cross-linking may be controlled in part by the molar ratio of cross-linking agent, methylene bis-acrylamide, to acrylamide.

A method for the preparation of a hydrogel comprises the steps of combining acrylamide and methylene bis-acrylamide, radical initiation, and washing with pyrogen-free water or saline solution so as to give less than 3.5% by weight polyacrylamide, based on the total weight of the polyacrylamide. The method is preferably such that the hydrogel comprises at least 0.5%, such as at least 1 %, preferably at least 1.5% polyacrylamide, such as at least 1.6% polyacrylamide by weight, based on the total weight of the hydrogel.

In a especially preferred embodiment of the invention, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution.

The combining of acrylamide and methylene bis-acrylamide in preferably done in a molar ratio between acrylamide and methylene bis-acrylamide is from 175:1 to 800:1, such as from 225:1 to 600:1, preferably from 250:1 to 550:1, most preferably from 250:1 to 500:1.

An illustrative preparation of the hydrogel according to the present invention is described in Example 1. The hydrogel having the desired physical properties has been obtained by combining acrylamide and methylene bis-acrylamide in a ratio of about 250:1, such as 252:1, 254:1, 256:1, 258:1 and 260:1, as well as by combining acrylamide and methylene bis-acrylamide in a ratio of about 500:1, such as 498:1, 496:1 494:1 492:1, or 490:1. The hydrogel according to the present invention preferably has a complex viscosity from about 2 to 90, such as 5 to 80 Pas, typically from about 6 to 76, such as from about 6 to 60, 6 to 40, 6 to 20, such as 6 to 15 Pas. In a suitable embodiment, the washing step comprises swelling the product of the radical initiation step until the complex viscosity is from about 6 to 100 Pas.

In a suitable embodiment of the invention, the hydrogel has a degree of cross-linking such that it has complex viscosity of not less than 2 Pas, such as not less than 3, 4 or 5 Pas, such as not less than 5.5 Pas, such as not less than 6 Pas, preferably not less than 6.2 Pas.

The elasticity module is an alternative physical characteristic of the hydrogel indicative, in part, of the degree of cross-linking of the hydrogel according to the present invention. Typically, the degree of cross-linking is such that the hydrogel has an elasticity module of not less than 10 Pa, such as not less than 25, 30, 31, 32, 33, 34 or 35 Pa, such as not less than 38 Pa. The gel may be characterised in that it has elasticity module from about 10 to 700 Pa, such as about 35 to 480 Pa.

As stated, in a most preferred embodiment, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution. The radical initiation step yields a hydrogel still comprising toxic reagents and not yet possessing the beneficial physical properties of the hydrogel of the present invention. The washing step comprises swelling the hydrogel produced from the radical initiation step until the complex viscosity is from about 2 to 90 Pas.

Alternatively measured, the washing step may comprise swelling the product of the radical initiation step until the elasticity module is elasticity module from about 10 to 700 Pa, such as from about 35 to 480 Pa.

By nature, a low degree of cross-linking typically results in a higher swelling rate consequently lowering the dry matter content (percentage acrylamide), as well as lowering the elasticity module and viscosity. Thus, in addition to the degree of cross-linking, the time the hydrogel is exposed to the washing step influences to a degree the physical properties of the gel.

Typically, the washing step comprises swelling the product for about 80 to 100 hours, such as 90-95 hours. This usually results in an increase in weight of the hydrogel of about 75 to 150%, usually around 100% increase.

Furthermore, the amounts of the free-radical initiator in the radical initiation step and the amount of co-initiator influences the chain length and thus the physical properties of the hydrogel. In a typical preparation of the hydrogel, N-,N-,N-,N-tetramethyl ethylene diamine (TMED) is used as the co-initiator and ammoniumpersulfate (APS) is used as the free-radical initiator (redox-system). Adequate amounts of initiator and co-initiator are required to obtain the hydrogel according to the invention. As an illustration, an insufficient amount of these reagents will result in shorter chain lengths and consequently influence the degree of cross-linking and hence the physical properties of the hydrogel. Other reaction conditions, such as temperature also influence chain length.

As stated, the degree of cross-linking influences the physical properties of the hydrogel. The degree of cross-linking of the hydrogel of the present invention may be indirectly measured, as described above, by the elasticity module and/or by the complex viscosity. An alternative measure of the degree of cross-linking of the hydrogel is its efficient cross-linking density. The hydrogel of the present invention preferably comprises 1.6 to 3.5% (wt/wt) polyacrylamide cross-linked with methylene bis-acrylamide and ii) pyrogen-free water or saline solution, preferably such that the degree of cross-linking as measured by its efficient cross-linking density is such that the efficient cross-linking density is about 0.2 to 0.5%, preferably about 0.25 to 0.4%.

In a suitable embodiment of the present invention, the hydrogel may comprise 1.6 to 3.25% (wt/wt) polyacrylamide, such as 1.8 to 3.1, 2.0 to 3.0, 2.0 to 2.9, preferably 2.0 to 2.8 (wt/wt) polyacrylamide.

### Medical Use of Polyacrylamide Hydrogels

The hydrogels of the invention are intended for use as endoprosthetic devices. The endoprosthetic devices of the invention and methods of treatment described herein may use the hydrogel in any of the embodiments described supra.

The endoprosthetic device may be administered to the body of an individual by means of injection, such as through a syringe or catheter or by means of surgical implantation. In the embodiment wherein the hydrogel is for use as an implantable endoprosthesis, the hydrogel may optionally serve as filler material in an envelope, the whole of which is implanted into the body. Thus the hydrogel or the endoprosthesis may comprise a silicone-based envelope housing the hydrogel.

The endoprosthetic device may comprise any of embodiments of the hydrogels discussed herein and may be either implantable or injectable Thus an important aspect of the present invention is the use of a hydrogel comprising i) less than 3.5% by weight polyacrylamide cross-linked with methylene bis-acrylamide and ii) at least 95% pyrogen-free water or saline solution for the preparation of an endoprosthesis for cosmetic surgery, reconstructive surgery and therapy. In particularly interesting embodiments of invention, the endoprosthesis is injectable.

Many disorders are related to a loss of effective activity of the tissue at a functional interface between two organs. For instance, urinary incontinence is related to insufficient sphincter between the urinary bladder and the urethra. By injecting or implanting an endoprosthesis prepared from the hydrogel according the present invention into the proximal submucosa of the urethra, thereby narrowing the urethra, the disorder may be significantly controlled. Similarly, reflux oesophagitis is related to insufficient resistance between the oesophagus and stomach. By injecting or implanting an endoprosthesis prepared from the hydrogel according the present invention along the sphincter between the oesophagus and stomach, the contact between the contents of the stomach and the oesophagus may be reduced.

The solid-weight content of weight percentage acrylamide, as measured after the washing step, as well as the degree of cross-linking is adapted according to the use of the endoprosthesis prepared from the hydrogel. In preferred embodiments, the endoprosthesis is preferably prepared from a hydrogel comprising 1.6 to 3.25% (wt/wt) polyacrylamide, such as 1.8 to 3.1, 2.0 to 3.0, 2.0 to 2.9, preferably 2.0 to 2.8 (wt/wt) polyacrylamide. Alternatively defined, the degree of cross-linking of the hydrogel for use as an endoprosthesis may preferably be such that the complex viscosity of the hydrogel is from about 2.0 to 15 Pas such as from about 5.5 to 15 Pas, such as from 6 to 12 Pas. Alternatively measured, for facial corrections the degree of cross-linking of the hydrogel are preferably such that the elasticity module is from about 10 to 100 Pa, such as about 35 to 75, particularly 35 to 60 Pa, such as 35 to 50 Pa.

The endoprosthesis may be to correct a defect which is the result of trauma such as tumours or physical injury as well as congenital defects.

In an alternative embodiment of the invention, the endoprosthesis may comprise a medicament for use in therapy.

### Polyacrylamide Hydrogel For The Treatment Of Arthritis

Arthritis is a degenerative condition which, when affecting the weight bearing joints such as the hip and knee joints, results in pain and hampered mobility. Arthritis may affect all joints. Degradation of articular and meniscal cartilage may result in damage to the surfaces separated by the cartilage and correspondingly to pain. Ageing is a primary cause of the degradation of the cartilage. The degradation may also result from, for example, congenital predisposition or trauma, such as repeated articulation of the joint.

Arthritis has been treated traditionally with physiotherapy and more invasive treatments such as orthopaedic surgery and the introduction of artificial joint components. Non-steroidal anti-inflammatory agents have been used with some success but these agents may counterproductively hamper proteoglycan synthesis in collagen and cartilage as well as have undesirable side effects. Cortisone injections also weaken articular cartilage with time.

Soft compliant materials used to replace the cartilage have been developed to absorb the load on the joint and to distribute the load evenly. US 4,344,193 dislcoses silicone rubber as a prosthetic device. One difficulty with these devices is securing the devices in place and various anchoring systems have been developed (US 5,171,322; US 4,502, 161; US 4,919,667).

Other prosthetic devices, such as in US 5,344,459, are inflatable. The Macintosh knee is a hard prosthetic which is painful to use.

WO 00/78356 discloses an injectable composition for promoting bone and/or cartilage growth comprising hyaluronic acid cross-linked to sulfated polysaccharides.

WO 96/24129 discloses a prosthesis for joints in hands and feet comprising a biocompatible material such as a mixture of biocompatible resin and plastics. Specific materials mentioned include polymethyl methacrylate polymer. The prosthesis is implanted into the joint. WO 00/59411 discloses a surgically implanted knee prosthesis wherein the load distribution device is constructed of material comprising a thermoset polymer or thermoplastic polymer. Hyaluronates and hyaluronic acids have been used for prosthetics and administered by injection into the intra-articular cavity of knees for the long-term relief of pain and improvement on the function of the knee joint. It has adequate viscosity and elasticity but has been prone to sheering due to mechanical stress and is biodegradable and has faced resorption problems.

There is the need in the art additional materials for use as an artificial cartilage such as in weight-bearing joints. The present invention is directed to a material and prosthetic device for use in the treatment of arthritis and to supplement or replace cartilage.

The polyacrylamide hydrogel is resistant to biological degradation and is not permeable through biological membranes. The polyacrylamide hydrogel of the invention is fully biocompatible (according to ISO standard test ISO-1 0993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. Notable for the invention, the polymer is resilient to mechanical stress.

The hydrogel of this aspect of the invention is for use in the in the treatment or prevention of arthritis, the hydrogel obtainable by combining acrylamide and methylene bis-acrylamide radical initiation; and washing with pyrogen-free water or saline solution, the combining being in amounts and the washing being such that to give about 0.5 to 25% by weight polyacrylamide, based on the total weight of the hydrogel. The hydrogel obtained has been shown by the present investigators to be both biostable and biocompatible, and is not resorbed by the body. Moreover, the present investigators have demonstrated the hydrogel to be resilient to mechanical stress.

Typically, the hydrogel of this aspect of the invention is obtained by combining acrylamide and methylene bis-acrylamide is in a molar ratio of 150:1 to 1000:1. The conditions for obtaining the hydrogel may be modified according to, for instance, the nature of the joint into which the hydrogel is intended to be injected. The desired rheologically properties, such as elasticity and viscosity may be controlled at least in part by the solid weight content of the hydrogel. The hydrogel of the invention comprises about 0.5 to 25% by weight polyacrylamide, based on the total weight of the hydrogel. In suitable embodiments of the invention, the hydrogel comprises less than 15% by weight polyacrylamide, based on the total weight of the hydrogel, preferably less 10%, more preferably less than 7.5%, even more preferably less than 5%, most preferably less than 3.5% by weight polyacrylamide, based on the total weight of the hydrogel.

Given the hydrogel of this aspect of the invention is directed for use as an endoprosthesis, it must be stable. Typically, such as for reasons of increased stability, the hydrogel comprises at least 1% by weight polyacrylamide, based on the total weight of the hydrogel, preferably at least 1.5%, such as 1.6% by weight polyacrylamide, based on the total weight of the hydrogel. In suitable embodiments, the hydrogel of the present invention has a solid weight content of at least 1.5 and less than 3.5% by weight polyacrylamide, based on the total weight of the hydrogel such as 1.5, 1.6, 1.7 1.8, 1.9, 2.0, 2.1, 2.2, 2.3, 2.4, 2.5, 2.6, 2.7, 2.8, 2.9, 3.0, 3.1, 3.2, 3.3, and 3.4% polyacrylamide, based on the total weight of the hydrogel.

The combining involves the combining of the component reagents acrylamide and methylene bis-acrylamide, typically degassed and typically in a manner to minimise operator contact. The reagent components may be optionally previously combined to form an inert mixture. An inert mixture is one wherein no chemical reaction proceeds among the component reagents. The combining involves combining acrylamide, methylene-bis-acrylamide, and a radical initiator component. In a suitable embodiment, an inert premixture of acrylamide, methylene-bis-acrylamide (the cross-linker) and TEMED is combined with an AMPS initiator solution. However, the components may be combined as singularities or as alternative plural premixtures.

Acrylamide and methylene-bis-acrylamide are suitably combined in a molar ratio of about 100:1 to 1000:1, typically about 150:1 to 900:1, preferably about 175:1 to 800:1, more preferably about 200:1 to 600:1, most preferably from 250:1 to 500:1. As shown in Tables 2 and 3, hydrogels of differing solid-weight content and rheological properties may be controllably prepared. The hydrogel having the desired rheological characteristics has been obtained by combining acrylamide and methylene-bis-acrylamide in a ratio of about 250:1, about 260:1, about 270:1, about 280:1, about 290:1, about about 300:1, about 310:1, about 320:1, about 330:1, about 340:1, about 350:1, about 360:1, about 370:1, about 380:1, about 390:1, about 400:1, about 410:1, about 420:1, about 430:1, about 440:1, about 450:1, about 460:1, about 470:1, about 480:1, about 490:1 and about 500:1.

As can also be seen from Tables 2 and 3, the relative amount of monomer (acrylamide and methylene-bis-acrylamide) is fairly constant from formulation to formulation in relation to TEMED. Thus, in a preferred embodiment of the method of the invention, the ratio of monomers to TEMED is relatively constant from batch to batch and not used to regulate the rheological properties of the polymer. In the embodiment wherein the polymer is polyacrylamide, the ratio of the monomers acrylamide and methylene-bis-acrylamide to TEMED is about 100:1 to 700:1, such as 200:1 to 600:1, typically 200:1 to 500:1, preferably 200:1 to 400:1, most preferably 200:1 to 350:1.

Similarly, the relative amount of monomer (acrylamide and methylene-bis-acrylamide) is fairly constant from formulation to formulation in relation to the amount of initiator. Thus, in a preferred embodiment of the method of the invention, the ratio of monomers to initiator is relatively constant from batch to batch and not used to regulate the rheological properties of the polymer. In the embodiment wherein the polymer is polyacrylamide, the ratio of the monomers acrylamide and methylene-bis-acrylamide to initiator is about 100:1 to 700:1, such as 200:1 to 600:1, typically 200:1 to 500:1, preferably 200:1 to 400:1, most preferably 200:1 to 350:1.

The viscosity of the hydrogel is suitably such that it may be injected. In a typical embodiment, the hydrogel has a complex viscosity of about 2 to 20 Pa s, such as about 3 to 18 Pa s, preferably about 3 to 15 Pa s, most preferably about 2 to 13 Pa s.

The hydrogel of the invention is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is a 0.8-1 % aqueous sodium chloride solution, such as a 0.8, 0.9 or 1 % aqueous sodium chloride solution, most preferably about 0.9 % aqueous sodium chloride.

As will be obvious to the person skilled in the art, in the embodiment wherein saline solution is used either for the preparation of the gel and/or for the washing of the gel, the solid-weight content of the gel will be higher than the contribution made by the polyacrylamide, but typically not more than an additional 1 %.

In a particularly suitable embodiment of the invention, the hydrogel comprises about 2.5 % by weight polyacrylamide, based on the total weight of the hydrogel and about 97.5 % pyrogen-free water.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, particularly preferably below 5 ppm.

In an alternative embodiment of the invention, the hydrogel has a more solid consistency such that it is implantable into a joint cavity. In such embodiments wherein the hydrogel has a solid consistency, the hydrogel may be surface-modified so as to decrease slippage from the area it was implanted. Surface modification may be chemical or physical in nature. Hydrogels of with a solid weight content have been prepared which are solid like and suitable for implantation rather than injection and very amenable to surface modification.

In the embodiment wherein the hydrogel is implantable, the viscosity of the solid sample is obviously very high. In the embodiment wherein the hydrogel is implantable, the hydrogel has a complex viscosity of about 20 to 1500 Pa s, typically 20 to 1000 Pa s.

In the embodiment of this aspect of the invention wherein the hydrogel is implantable and furthermore surface-modified, in the event that the hydrogel is surface modified by chemical treatment, it is anticipated that the chemical treatment will account for less than 1 % of the weight of the hydrogel based on the total weight of the hydrogel. Chemical treatment may comprise of a surface coating agent or an agent which acts topically to chemically modify the polyacrylamide at the surface of the hydrogel.

The hydrogel of this aspect of the invention is used for the preparation of an endoprosthesis to be inserted into the intra-articular cavity of joint. The gel is intended for use as a prosthetic device in the treatment of arthritis. The prosthetic device of the invention comprises a polyacrylamide hydrogel comprising 0.5 to 25% by weight polyacrylamide, based on the total weight of the hydrogel and is administered to the intra-articular cavity of joint.

The prosthetic device may comprise any embodiment of the hydrogel as described supra. According, the device is for augmenting or replacing cartilage in the intra-articular cavity of a joint, said device comprises a polyacrylamide hydrogel comprising 0.5 to 25% by weight polyacrylamide, based on the total weight of the hydrogel. The prosthetic device of the invention the hydrogel typically further comprises at least 75% by weight pyrogen-free water or saline solution, preferably pyrogen-free water. It may be administered by implantation or injection into the intra-articular cavity of a joint. Preferably, the device is injected.

The device may have a viscosity such that it may be injected. In an embodiment wherein the hydrogel is injected, the hydrogel has a complex viscosity of about 2 to 25 Pa s, such as about 3 to 20 Pa s, preferably about 3 to 18 Pa s, most preferably about 3 to 15 Pa s, such as 3, 4, 5, 6, 7, 8, 9, 10, 11, 12, 13, 14, and 15 Pa s.

Particularly in the embodiment wherein the hydrogel is injected into a weight-bearing joint, the elasticity of the hydrogel and device is of great relevance. In a preferred embodiment, the hydrogel of the invention has an elasticity modulus of about 1 to 200 Pa, such as about 2 to 175 Pa, typically about 5 to 150 Pa, such as 10 to 100 Pa.

The elasticity modulus of the prosthetic device and the complex viscosity are typically related by a factor of 5.8 to 6.4. The present invention thus provides for a hydrogel with the advantageous combined features of a viscosity suitable for being injectable and of an elasticity to supplement weight-bearing capacity. In a combination of preferred embodiments, the hydrogel has a complex viscosity less than 25 Pa s and an elasticity modulus less than 200 Pa, preferably having a complex viscosity less than 15 Pa s and an elasticity modulus less than 100 Pa.

Example 1, Tables 1, 2 and 3, illustrate appropriate conditions for preparing the illustrative examples of hydrogels. As can be seen, within the preferred combined embodiment of a viscosity of less than 25 Pa s and an elasticity modulus of less than 200 Pa, such as a complex viscosity of less than 15 Pa s and an elasticity of less than 100, the hydrogel may have an array of dry-weight content percentages.

Furthermore, still within the preferred combined embodiment of a viscosity of less than 25 Pa s and an elasticity less than 200 Pa, such as having a complex viscosity less than 15 Pa s and an elasticity less than 100, the hydrogel is obtainable by combining acrylamide and methylene-bis-acrylamide in a molar ratio of about 275 to 1000, typically 300 to 800, preferably in a ratio of about 300 to 500.

The hydrogel of the invention is resilient to mechanical stress as well as prividing lubrication within the joint. This mimics the combination of naturally occurring cartilage and synovial fluid in the joint. Synovial fluid is produced by the synovial membrane and forms in the interface with both the synovium and the articular cartilage. Its function is nutrition of the cartilage, lubrication, load bearing and shock absorption (see Gomez and Thurston, Biorheology 30, 409-427 (1993)). Synovial liquid is a solution of a very sophisticated polymer complex made of the linear hyaluronic acid backbone having protein branches and has an elasticity modulus G' of 60 Pa and a complex viscosity of about 1-10 Pa s. Certain embodiments of the hydrogel of the invention typically has very similar elasticity modulus and viscosity.

Synovial liquid, being a fluid, has a very long relaxation time (about 100 sec). The relaxation time is defined as the time required for the stress to decay to 37% of its initial value in a stress relaxation experiment. This long relaxation time implies that for fast movements (when the stress is applied rapidly), it responds as a very elastic material, whereas at low speed stresses it behaves like a lubricating oil. Low viscosity formulations of the hydrogel of the invention are fluid-like, such as when homogenised, and as fluids, have the very long relaxation time and the fluid like properties of synovial liquid. The present invention thus provides for an excellent alternative to known technologies for replacing synovial liquid, such as injections with isotonic (NaCl) water which lasts for only 1-3 days, or injection with solutions of hyaluronic acid, which faces resorption problems.

As stated, in embodiments wherein the hydrogel is as a low viscosity formulation, the hydrogel attempts to mimic, at least in part, the features of synovial liquid. In embodiments where the hydrogel is of higher viscosity formulation, such as a viscosity above 10 Pa s, such as above 15 Pa s, the hydrogel mimics more the combined features of synovial liquid and cartilage in that the hydrogels are more elastic materials with infinite relaxation times.

In terms of resilience to mechanical stress, the hydrogel of the invention has demonstrated complete full resilient behaviour. Moreover, the elastic feature of the hydrogel allows it to return to its start position when the stress is released in creep experiments.

The device may be administered into an array of intra-articular cavities where said joint or cartilage present in said joint may need increased lubrication, increased weight bearing capacity, or increased protection of the opposing bones of the joint, such as but not limited to the group comprising of the knee joint; hip joint; the elbow, the metacarpal-phalangeal and interphalangeal joints in hands and feet (please comment as to other relevant joints).

In the administration of the device to the patient, the adequate placing and position of the hydrogel is of great relevance. To assist in the positioning of the gel, visualisation of the location of the device is useful to the individual performing the procedure. It may be advantageous, such as in the embodiment wherein the device is administered by injection to visualise the device in order to establish its position and the amount required. Visualisation of the hydrogel during administration may be facilitated by radio-labelling of the hydrogel. Thus, in one interesting embodiment, the hydrogel is radio-labelled.

As stated, surface treatment of the hydrogel may assist device to be held in place within the joint cavity. However, in the embodiment of an injectable hydrogel, the device may be held by the bone tissue which defines the boundaries of the cavity into which the gel was injected.

As also stated, one aspect of the invention relates to the use of a hydrogel comprising about 0.5 to 25% by weight polyacrylamide, based on the total weight of the hydrogel, for the preparation of an endoprosthesis for alleviation or prevention of symptoms associated with arthritis. Correspondingly, the method of the invention may be defined as a method of treating or preventing arthritis comprising administering a hydrogel to a mammal said hydrogel comprising 0.5 to 25% by weight polyacrylamide, based on the total weight of the hydrogel. The method comprises the use of the hydrogel as defined supra and to the use of the prosthetic device as defined supra.

The method may comprise a first series or treatment session of injections or implantations followed by an analysis, evaluation or trial of the degree of assistance provided by the device and followed by further series or treatment sessions if required. In the embodiment wherein the joint required primarily increased weight bearing capacity, the elasticity or support provided by the device may diminish with time. However, one advantage of the method of the invention is that an injectable formulation of the prosthetic device may be administered to supplement the existing device. This procedure may be repeated as often as required by the patient to alleviate the pain associated with the condition. Similarly, in the event that the device was administered primarily to increase lubrication in the joint, and that the lubrication capacity of the device has diminished with time, the method of the invention, such as by simple injection may be repeated as required.

### EXAMPLES

### Example 1

### Preparation of Hydrogel

The gel is a polyacrylamide gel manufactured by a polymerisation of the monomers of acrylamide and N,N'-methylene-bis-acrylamide. The finished product may have different viscosities.

The hydrogel has the empirical formula [C₃H₅NO]ₓ[C₇H₁₀N₂O₂]_{y} and the structural formula as shown in Figure 1

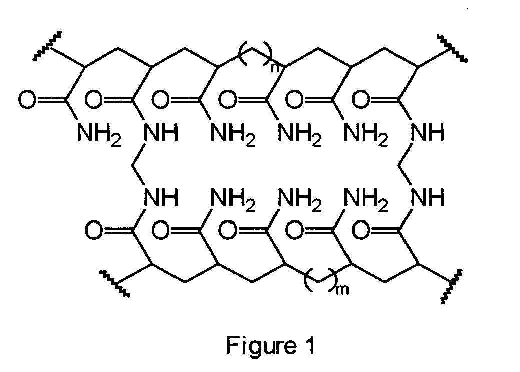

The hydrogel typically contains approximately 95% water. The concentration of the monomers acrylamide and N, N'-methylene-bis-acrylamide has been shown to be less than 10 ppm and is adequate for the desired stability of the final product, often less than 5 ppm.

The finished product must conform with respect to pH, absence of heavy metals, refractive index, stability, absence of pyrogens, and must be sterile, practically inert, and be substantially free of monomers.

### Preparation 1.1

The synthetic preparation suitably involves the following operations:
1. Two mixtures, A1 and A2, are prepared. A1 comprises water, acrylamide, N,N'-methylene-bis-acrylamide, N,N,N',N'-tetramethylene-ethylene-diamine (TEMED). A2 comprises water and ammonium persulphate;
2. The two mixtures are combined in the following ratio: 1990 mL of A1 and 10 mL of A2 and kept at 45 °C and degassed with nitrogen for 20 seconds;
3. The reaction mixture is cast into several 100 mL beakers;
4. Polymerisation is allowed to occur for 0.5 to 1.5 hours;
5. The gel is demolded;
6. Residual monomers are extracted and with equilibration in WFI water for 92 hours, changing the water several times, typically 8 times during the 92 hours;
7. The purified gels are homogenised by grinding with an vertically oscillating grid;
8.The syringe is filled with the homogenised gel material;
9. Autoclavation of the syringe

A typical method for preparing the hydrogel may be summarised as:

### Preparation 1.2

*Process summary.* The gel is prepared by mixing an aqueous monomer solution of acrylamide (AM) and N,N'-methylene-bis-acrylamide (BISAM) as cross-linker with N,N,N',N'-tetramethylene ethylene diamine (TMED) as co-initiator and ammoniumpersulfate (APS) as free-radical initiator (redox-system). By degassing a bulk solution with nitrogen polymerisation starts. After final polymerisation the gel transferred into a washing tank with net trays onto which the gel is placed. During water washing the gel swells and monomer residues are extracted. The swollen gel is fed and evacuated in a filling unit having the gel delivered in a syringe, which is autoclaved.

Two alternate formulations have been prepared, a lower- and a higher- end viscosity formulation. Both formulations have a solid weight content of less than 3.5% and a complex viscosity in the range of 2 to 50 Pa s, typically between 3 and 20 Pa s.

**Table 1**

| Chemical constituent | lower end viscosity | higher end viscosity |
|---|---|---|
| acrylamide | 502 g | 547 g |
| N,N'-methylene-bis-acrylamide | 2,2 g | 4,6 g |
| TMED | 3,0 g | 2,6 g |
| APS | 5,4 g | 5,0 g |
| Non-pyrogenic water | Add 10 litre | Add 10 litre |

The above are typical preparations of the hydrogel and may be adjusted within certain ranges.

### Preparation 1.3

### Polyacrylamide formulations from inline cross-linking process

A particularly interesting method of preparing the hydrogels of the invention involves an inline cross-linking process. Two individual and eventually degassed flows, one being a pre-mix of acrylic amide, bis-methylene acryl amide (the cross-linker) and TEMED, the other being the AMPS initiator solution, are pumped into a static mixer for mixing, chemical initiation and subsequent extrusion downstream into a pipe reactor made of Teflon or steel in which the polymerisation occurs. Washing of the gel is simplified due to high surface area of gel from reactor.

By selecting monomer, cross-linker and initiator concentrations and their relative molar ratios, and by regulating the two flow rates and the polymerisation temperatures, it is possible to produce gels that are varying in degree of crosslinking and in solids content.

### Preparation 1.4

The reagents were combined in ratios described in Tables 2, 3 and 4, and washed as described in the Tables (with pyrogen-free water unless indicated otherwise) to give low, medium, and high viscosity formulations. Hydrogels with solid weight contents between 0.5 and 25% polyacrylamide were prepared.

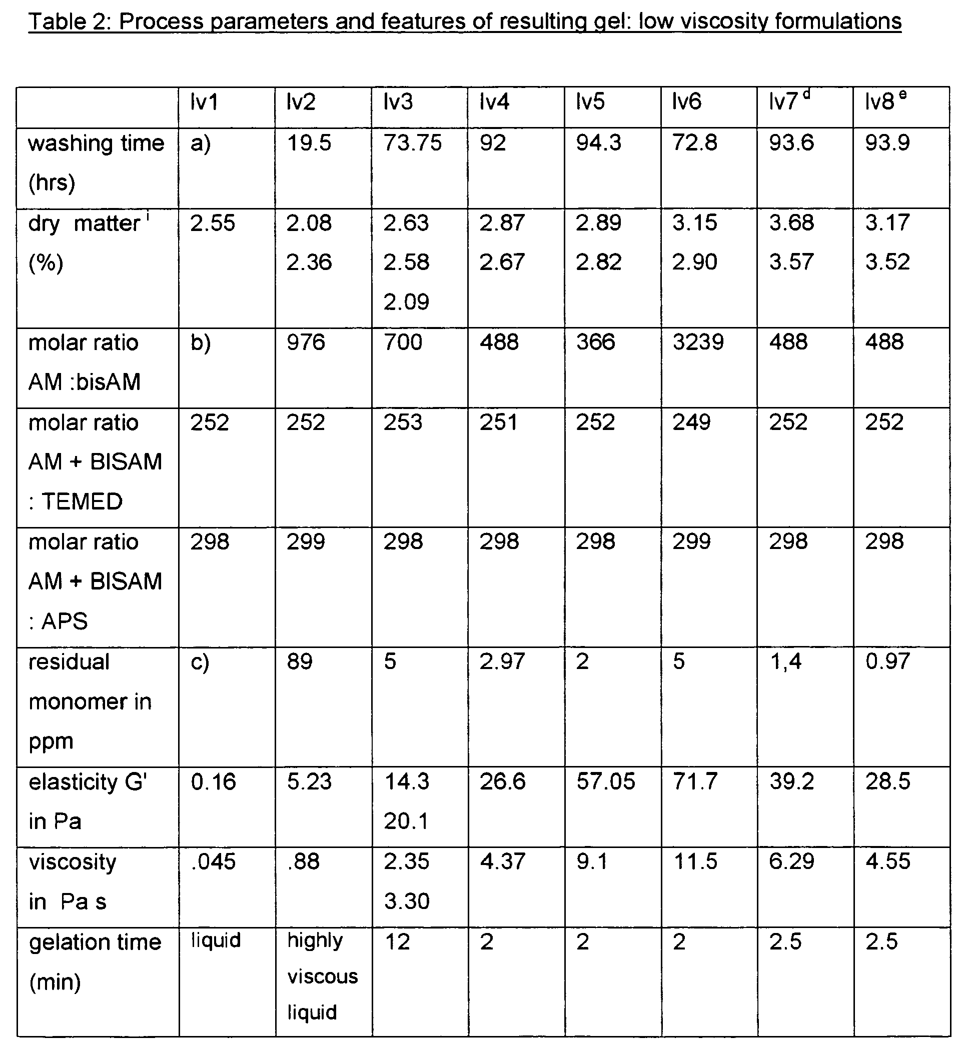

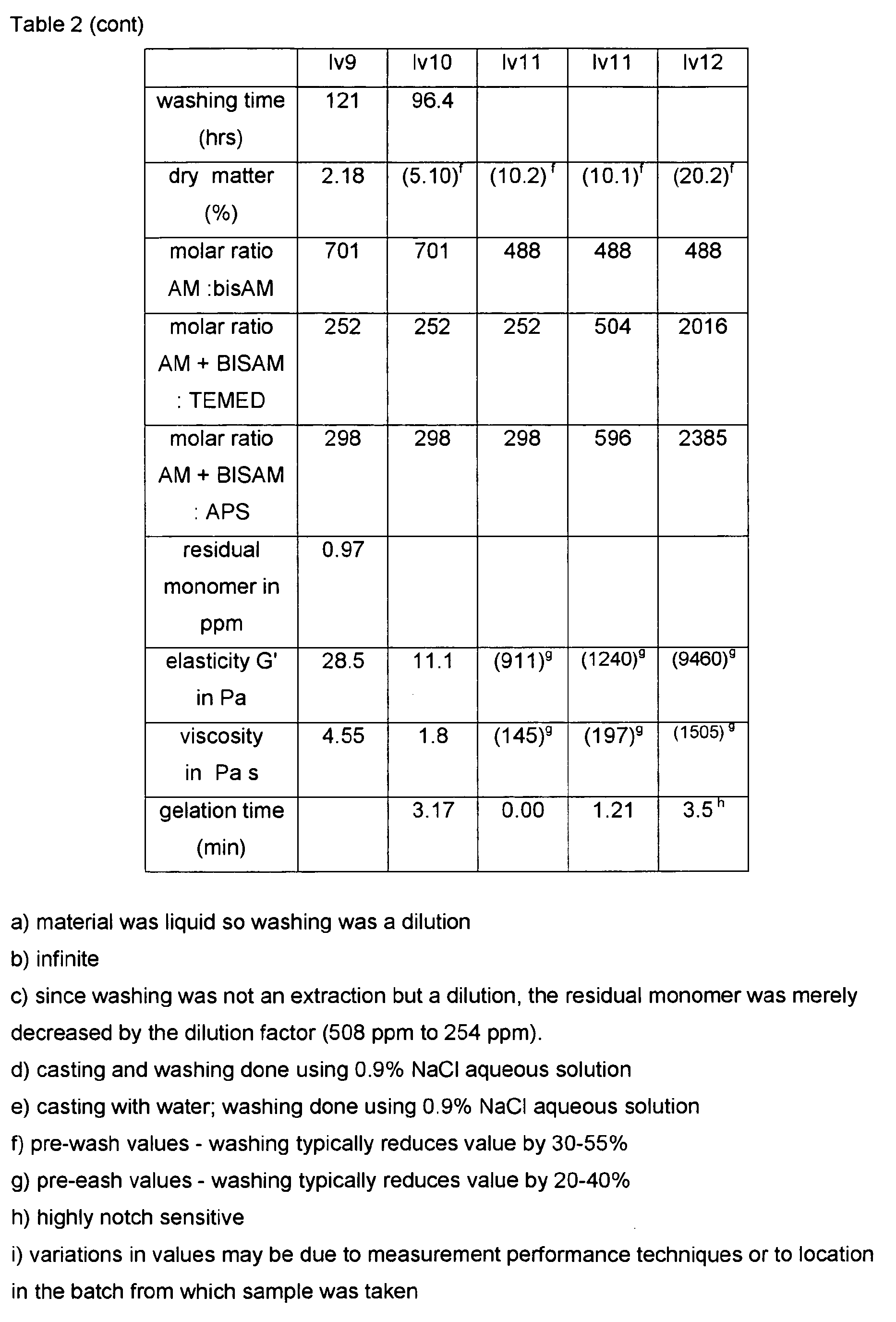

**Table 3: Process parameters and features of resulting gel: medium viscosity formulations**

| | mv1 | mv2 | mv3 | mv4 | mv5 |
|---|---|---|---|---|---|
| washing time (hrs) | 97 | 211.5 | 96 | 94.8 | 90.3 |
| dry matter (%) | 3.14 | 2.49 | 3.25 | 3.29 | 3.22 |
| molar ratio AM :bisAM | 310 | 310 | 290 | 289 | 289 |
| molar ratio AM + BISAM : TEMED | 252 | 252 | 252 | 251 | 252 |
| molar ratio AM + BISAM : APS | 299 | 299 | 299 | 299 | 299 |
| residual monomer in ppm | 1.6 | | 1.5 | | |
| elasticity G' in Pa | 108.5 | | 129 | 133.5 | |
| viscosity in Pa s | 17.4 | | 20.6 | 21.30 | |
| gelation time (min) | 2.5 | 2.5 | 2.18 | | |

**Table 4: Process parameters and features of resulting gel: high viscosity formulations**

| | hv1 | hv2 | hv3 | hv4 | hv5 |
|---|---|---|---|---|---|
| washing time (hrs) | 119.5 | 516 | 122 | 95.5 | 116.7 |
| dry matter (%) | 3.47 | 2.5 | 3.56 | 3.83 | 3.42 |
| molar ratio AM :bisAM | 260 | 260 | 260 | 260 | 260 |
| molar ratio AM + BISAM : TEMED | 315 | 315 | 604 | 313 | 314 |
| molar ratio AM + BISAM :APS | 376 | 376 | 755 | 375 | 376 |
| residual monomer in ppm | 0.2 | | | | |
| elasticity G' in Pa | 343 | 274 | | 314.5 | |
| viscosity in Pa s | 54.7 | 43.65 | | 50.1 | |
| gelation time (min) | 2.18 | 2.18 | 7.5 | | |

### Example 2

### Analysis of Hydrogel

### Characteristics of the gel

The washed hydrogels will have typical properties outlined below.

**Table 5**

| Property | Low viscosity version | High viscosity version |
|---|---|---|
| Cross-linking density | 0,25% | 0,40% |
| Swelling rate | 80-120% | 50-70% |
| Rheol. elasticity modulus, G' | 10-80 Pa | 250-700 Pa |
| Rheol. Complex viscosity, η* | 2-10 Pas | 50-90 Pas |
| Dry matter | 1,6-3% | 3-5% |
| Refractive index | 1,335-1,338 | 1,338-1,340 |

### Example 3

Swelling occurs during wash procedure and will typically show a swelling profile as shown in below Figures 2 and 3.

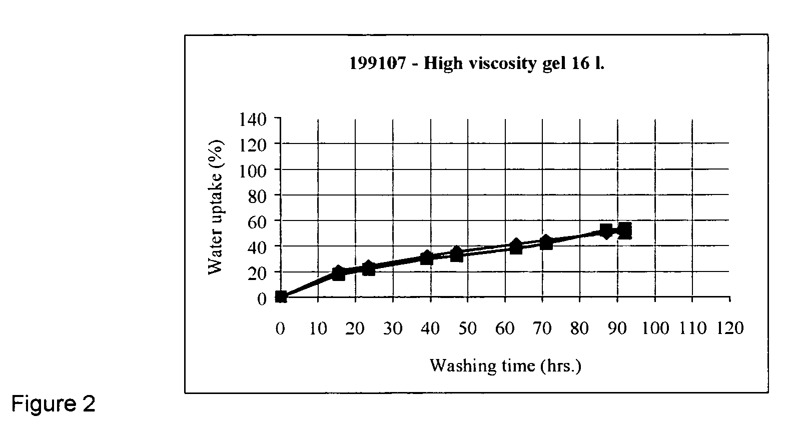

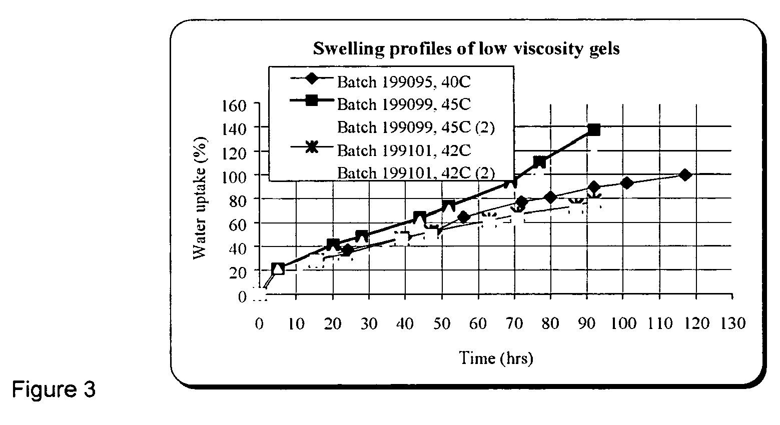

## Claims

1. A hydrogel for use in the alleviation or prevention of symptoms associated with arthritis,
said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

2. A hydrogel for use in augmenting or replacing cartilage in the intra-articular cavity of a joint,
said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

3. A hydrogel for use in providing lubrication to a joint and the pre-existing cartilage, said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

4. The hydrogel according to claim 3, wherein the joint or cartilage present in said joint is in need of increased lubrication, increased weight bearing capacity, and/or increased protection of the opposing bones of the joint.

5. The hydrogel according to claim 3 or 4, wherein the joint is selected from the group comprising of the knee joint, hip joint, the elbow, and the metacarpal-phalangeal and interphalangeal joints in hands and feet.

6. The hydrogel according to claim 1, wherein the arthritis is in the knee joint, hip joint, the elbow, or metacarpal-phalangeal and interphalangeal joints in hands and feet.

7. The hydrogel according to any one of the preceding claims, wherein the hydrogel has an elasticity module of from 10 to 700 Pa.

8. The hydrogel according to any one of the preceding claims, wherein the hydrogel has an elasticity module of from 35 to 480 Pa.

9. The hydrogel according to any one of the preceding claims, wherein the acrylamide and methylene bis-acrylamide are combined in a molar ratio of 150:1 to 1000:1.

10. The hydrogel according to claim 9, wherein the acrylamide and methylene bis-acrylamide are combined in a molar ratio of 200:1 to 600: 1.

11. The hydrogel according to any one of the preceding claims comprising less than 15% by weight polyacrylamide.

12. The hydrogel according to claim 11 comprising less than 7.5% by weight polyacrylamide.

13. The hydrogel according to claim 12 comprising 0.5 to 3.5% by weight polyacrylamide.

## Patentansprüche

1. Hydrogel zur Verwendung bei der Linderung oder Prävention von mit Arthritis verbundenen Symptomen,
wobei das Hydrogel durch Vereinigen von Acrylamid und Methylenbisacrylamid in Mengen, die etwa 0,5 bis 25 Gew.-% Polyacrylamid bezogen auf das Gesamtgewicht des Hydrogels ergeben, Radikalinitiierung und Waschen mit pyrogenfreiem Wasser oder Kochsalzlösung erhältlich ist.

2. Hydrogel zur Verwendung bei der Verstärkung oder dem Ersatz von Knorpel in der Gelenkhöhle eines Gelenks,
wobei das Hydrogel durch Vereinigen von Acrylamid und Methylenbisacrylamid in Mengen, die etwa 0,5 bis 25 Gew.-% Polyacrylamid bezogen auf das Gesamtgewicht des Hydrogels ergeben, Radikalinitiierung und Waschen mit pyrogenfreiem Wasser oder Kochsalzlösung erhältlich ist.

3. Hydrogel zur Verwendung bei der Schmierung eines Gelenks und bereits vorhandenem Knorpel,
wobei das Hydrogel durch Vereinigen von Acrylamid und Methylenbisacrylamid in Mengen, die etwa 0,5 bis 25 Gew.-% Polyacrylamid bezogen auf das Gesamtgewicht des Hydrogels ergeben, Radikalinitiierung und Waschen mit pyrogenfreiem Wasser oder Kochsalzlösung erhältlich ist.

4. Hydrogel nach Anspruch 3, wobei das Gelenk oder der im Gelenk vorhandene Knorpel vermehrte Schmierung, ein höheres Gewichtsbelastungsvermögen und/oder erhöhten Schutz der dem Gelenk gegenüberliegenden Knochen benötigt.

5. Hydrogel nach Anspruch 3 oder 4, wobei das Gelenk ausgewählt ist aus der Gruppe, umfassend das Kniegelenk, das Hüftgelenk, den Ellbogen und die metakarpal-phangealen und interphangealen Gelenke an Händen und Füßen.

6. Hydrogel nach Anspruch 1, wobei die Arthritis im Kniegelenk, dem Hüftgelenk, dem Ellbogen oder den metakarpal-phangealen und interphangealen Gelenken an Händen und Füßen ist.

7. Hydrogel nach einem der vorhergehenden Ansprüche, wobei das Hydrogel ein Elastizitätsmodul von 10 bis 700 Pa aufweist.

8. Hydrogel nach einem der vorhergehenden Ansprüche, wobei das Hydrogel ein Elastizitätsmodul von 35 bis 480 Pa aufweist.

9. Hydrogel nach einem der vorhergehenden Ansprüche, wobei das Acrylamid und das Methylenbisacrylamid in einem molaren Verhältnis von 150:1 bis 1000:1 vereinigt werden.

10. Hydrogel nach Anspruch 9, wobei das Acrylamid und das Methylenbisacrylamid in einem molaren Verhältnis von 200:1 bis 600:1 vereinigt werden.

11. Hydrogel nach einem der vorhergehenden Ansprüche, wobei es weniger als 15 Gew.-% Polyacrylamid umfasst.

12. Hydrogel nach Anspruch 11, wobei es weniger als 7,5 Gew.-% Polyacrylamid umfasst.

13. Hydrogel nach Anspruch 12, wobei es 0,5-3,5 Gew.-% Polyacrylamid umfasst.

## Revendications

1. Hydrogel destiné à être utilisé pour le soulagement ou la prévention des symptômes associés à l'arthrite,
ledit hydrogel pouvant être obtenu par combinaison d'acrylamide et de méthylènebisacrylamide dans des quantités telles que l'on obtient environ 0,5 à 25 % de polyacrylamide en poids, sur la base du poids total de l'hydrogel ; initiation radicalaire et lavage avec de l'eau apyrogène ou une solution saline.

2. Hydrogel destiné à être utilisé pour compléter ou remplacer du cartilage dans la cavité articulaire d'une articulation,
ledit hydrogel pouvant être obtenu par combinaison d'acrylamide et de méthylènebisacrylamide dans des quantités telles que l'on obtient environ 0,5 à 25 % de polyacrylamide en poids, sur la base du poids total de l'hydrogel ; initiation radicalaire et lavage avec de l'eau apyrogène ou une solution saline.

3. Hydrogel destiné à être utilisé pour lubrifier une articulation et le cartilage préexistant,
ledit hydrogel pouvant être obtenu par combinaison d'acrylamide et de méthylènebisacrylamide dans des quantités telles que l'on obtient environ 0,5 à 25 % de polyacrylamide en poids, sur la base du poids total de l'hydrogel ; initiation radicalaire et lavage avec de l'eau apyrogène ou une solution saline.

4. L'hydrogel selon la revendication 3, dans lequel l'articulation ou le cartilage présent dans ladite articulation nécessite une lubrification plus importante, une capacité de mise en charge plus importante et/ou une protection plus importante des os opposés de l'articulation.

5. L'hydrogel selon la revendication 3 ou 4, dans lequel l'articulation est choisie dans le groupe comprenant l'articulation du genou, l'articulation de la hanche, le coude et les articulations métacarpophalangiennes et interphalangiennes des mains et des pieds.

6. L'hydrogel selon la revendication 1, dans lequel l'arthrite se situe au niveau de l'articulation du genou, de l'articulation de la hanche, du coude ou des articulations métacarpophalangiennes et interphalangiennes des mains et des pieds.

7. L'hydrogel selon l'une quelconque des revendications précédentes, dans lequel l'hydrogel présente un module d'élasticité de 10 à 700 Pa.

8. L'hydrogel selon l'une quelconque des revendications précédentes, dans lequel l'hydrogel présente un module d'élasticité de 35 à 480 Pa.

9. L'hydrogel selon l'une quelconque des revendications précédentes, dans lequel l'acrylamide et le méthylènebisacrylamide sont combinés dans un rapport molaire de 150/1 à 1000/1.

10. L'hydrogel selon la revendication 9, dans lequel l'acrylamide et le méthylènebisacrylamide sont combinés dans un rapport molaire de 200/1 à 600/1.

11. L'hydrogel selon l'une quelconque des revendications précédentes, comprenant moins de 15 % de polyacrylamide en poids.

12. L'hydrogel selon la revendication 11, comprenant moins de 7,5 % de polyacrylamide en poids.

13. L'hydrogel selon la revendication 12, comprenant 0,5 à 3,5 % de polyacrylamide en poids.
